# EUROPÄISCHE PATENTSCHRIFT

(11) **EP 3 668 405 B1**
(45) Veröffentlichungstag und Bekanntmachung des Hinweises auf die Patenterteilung: **13.01.2021**
(21) Anmeldenummer: 18759896.6
(22) Anmeldetag: 17.08.2018
(51) Int. Cl.: A61B 6/03, A61B 6/04, A61B 6/00

(54) **COMPUTERTOMOGRAPHIEVORRICHTUNG**
COMPUTER TOMOGRAPHY APPARATUS
DISPOSITIF DE TOMODENSITOMÉTRIE

(30) Priorität: 17.08.2017 DE 102017007722
(43) Veröffentlichungstag der Anmeldung: 24.06.2020
(73) Patentinhaber: Schulze, Thorben, 12165 Berlin (DE)
(72) Erfinder: Schulze, Thorben, 12165 Berlin (DE)
(74) Vertreter: Eisenführ Speiser
(86) Internationale Anmeldenummer: PCT/EP2018/072372
(87) Internationale Veröffentlichungsnummer: WO 2019/034793

(56) Entgegenhaltungen:
- EP-A1- 0 387 956
- US-A1- 2016 128 653
- Jimmy Saunders Alastair Nelson Katrien Vanderperren: "Particularities of Equine CT" In: Eds T. Schwarz and J. Saunders: "Veterinary Computed Tomography", 23. August 2013 (2013-08-23), John Wiley & Sons, XP002786643, Seiten 421-426, DOI: 10.1002/9781118785676.ch39, Seite 421 - Seite 423; Abbildungen

## Beschreibung

Die Erfindung betrifft eine Computertomographievorrichtung zum Untersuchen eines Körperteils eines Großtieres und ein Verfahren zum Untersuchen eines Körperteils eines Großtieres mit einer Computertomographievorrichtung.

Ein Computertomographie (CT)-Gerät weist typischerweise eine als Ringtunnel ausgebildete Gantry auf sowie einen in die mittige Öffnung der ringförmigen Gantry einfahrbaren CT-Tisch, auf dem ein Patient oder ein Untersuchungsobjekt gelegt werden kann. In der Gantry sind eine CT-Röhre und der CT-Röhre gegenüber ein Detektorsystem exzentrisch angeordnet, die sich während einer CT-Untersuchung in dem Ringtunnel drehen. Dabei emittiert die CT-Röhre einen Fächerstrahl aus Röntgenstrahlen in Richtung des Detektorsystems. Wenn ein Patient oder ein Untersuchungsobjekt auf dem CT-Tisch in die mittige Öffnung der ringförmigen Gantry eingefahren ist, durchdringen die Röntgenstrahlen das zu untersuchende Gebiet und werden in ihrer Intensität abhängig von den Absorptionseigenschaften innerhalb des beleuchteten Gebiets abgeschwächt. Wenn sich in dem beleuchteten Gebiet Strukturen mit unterschiedlichen Absorptionseigenschaften befinden, beispielsweise Knochen- und Weichteilgewebe, werden die Röntgenstrahlen in dem beleuchteten Gebiet unterschiedlich stark abgeschwächt. Der Detektor detektiert eine zweidimensionale Projektion des in der Regel dreidimensionalen beleuchteten Objekts, wobei die Projektion eine Intensitätskarte der detektierten Röntgenstrahlen repräsentiert. Durch die Drehung der CT-Röhre und des Detektorsystems können während einer CT-Untersuchung eine Vielzahl Projektionen eines zu untersuchenden Objekts aus verschiedenen Richtungen aufgenommen werden, aus denen dann rechnerisch durch einen Computer die ursprüngliche dreidimensionale Struktur des untersuchten Objekts rekonstruiert werden kann.

Jedoch ist ein eingangs beschriebenes handelsübliches CT-Gerät nur begrenzt geeignet, eine CT-Untersuchung an einem stehenden Großtier durchzuführen. Oftmals kann eine CT-Untersuchung an einem liegenden Großtier nur in Vollnarkose durchgeführt werden und ist deshalb vergleichsweise riskant und aufwendig. CT-Geräte, mit denen eine CT-Untersuchung an einem stehenden Großtier möglich ist, sind oftmals auf die Untersuchung von bestimmten Körperteilen eines Großtieres eingeschränkt. Wenn beispielsweise für eine CT-Untersuchung eine auf Schienen verfahrbare Gantry eines CT-Geräts (engl. *Sliding Gantry*) verwendet wird, können bei einem stehenden Großtier, beispielweise einem Pferd, typischerweise nur der Kopf und der Hals und insbesondere nicht die Gliedmaßen untersucht werden. Ein solches Gerät, mit dem der Kopfbereich eines stehenden Pferdes untersucht werden kann, wird beispielsweise in der US-A1-2016/128653 beschrieben. Bei einer solchen Anordnung einer Gantry auf Schienen kann es erforderlich sein, einen Patienten auf einer Hebebühne stehend auf eine bestimmte Höhe anzuheben, damit ein zu untersuchendes Körperteil in der Öffnung der ringförmigen Gantry zu positionieren. Ein Großtier, beispielsweise ein Pferd, auf einer Hebebühne anzuheben stellt ein erhebliches Risiko für das Großtier dar und erschwert eine CT-Untersuchung an einem Großtier zusätzlich, da beispielsweise ein Großtier in der Regel nur langsam angehoben werden kann, wodurch sich eine Untersuchungszeit deutlich verlängert. Eine Anordnung einer Gantry auf Schienen benötigt zudem im Vergleich zu einem eingangs beschriebenen CT-Gerät wesentlich mehr Platz. Beide eingangs beschriebenen Varianten weisen also eine Reihe von Nachteilen auf.

In US 9,301,726 B2 wird eine CT-Maschine zum Scannen eines stationären Patienten, beispielsweise einen Menschen oder ein Pferd, beschrieben. Die CT-Maschine weist Gelenkarme mit zwei Gelenkstangen auf, um eine CT-Gantry auf einer beliebigen Trajektorie zu bewegen und unterscheidet sich somit deutlich von dem grundlegenden Aufbau eines eingangs beschriebenen, handelsüblichen CT-Geräts.

Es ist eine Aufgabe der Erfindung eine verbesserte Computertomographievorrichtung zum Untersuchen eines Körperteils eines stationär stehenden Großtieres sowie ein verbessertes Verfahren zum Untersuchen eines Körperteils eines stationär stehenden Großtieres mit einer Computertomographievorrichtung bereitzustellen.

Hinsichtlich der Computertomographievorrichtung wird die Aufgabe der Erfindung gelöst durch eine Computertomographievorrichtung zum Untersuchen eines Körperteils eines Großtieres aufweisend ein Computertomographie (CT)-Gerät und eine Plattform, auf der das CT-Gerät montiert ist. Das CT-Gerät umfasst eine ringförmige Gantry und einen CT-Tisch zur Aufnahme eines zu untersuchenden Körperteils eines Großtieres. Die Gantry ist relativ zu der Plattform auf der Plattform angeordnet.

Der CT-Tisch ist relativ zu der Plattform und zu der Gantry horizontal beweglich mit der Gantry und/oder der Plattform derart verbunden , sodass der CT-Tisch in eine mittige Öffnung der ringförmigen Gantry eingefahren werden kann.

Erfindungsgemäß umfasst die Computertomographievorrichtung weiterhin eine Verschiebevorrichtung, die mit der Plattform verbunden und ausgebildet ist, die Plattform mit dem montierten CT-Gerät relativ zu einer Standfläche eines Großtieres horizontal zu verschieben. Die Verschiebevorrichtung ist weiterhin ausgebildet, in einem Betriebszustand die Plattform mit dem montierten CT-Gerät derart zu verschieben, dass der CT-Tisch während einer Relativbewegung zur Gantry relativ zu der Standfläche stationär bleibt.

Im Rahmen dieser Beschreibung wird unter einer Standfläche ein fester Untergrund verstanden, der sich auf derjenigen Seite der Gantry befindet, die dem CT-Tisch gegenüberliegt. Die Standfläche befindet sich außerhalb der Computertomographievorrichtung. Während einer CT-Untersuchung mit der erfindungsgemäßen Computertomographievorrichtung steht ein zu untersuchendes Großtier stationär auf der Standfläche.

Das CT-Gerät der erfindungsgemäßen Computertomographievorrichtung ist wie ein handelsübliches CT-Gerät aufgebaut und umfasst eine Gantry und einen CT-Tisch zur Aufnahme eines zu untersuchenden Körperteils eines Großtieres. Für eine CT-Untersuchung kann der CT-Tisch in die Gantry eingefahren werden, um so wie eingangs beschrieben Projektionen aus verschiedenen Richtung aufzunehmen, die computergestützt verarbeitet werden können. Vorteilhafterweise muss also an dem CT-Gerät der erfindungsgemäßen Computertomographievorrichtung keine wesentliche Veränderung im Vergleich zu einem handelsüblichen CT-Gerät vorgenommen werden.

Der Erfindung liegt nun die Erkenntnis zugrunde, dass es vorteilhaft ist, ein handelsübliches CT-Gerät zu verwenden, da sich so der Aufwand eines Umbaus oder der Aufwand einer Neuentwicklung eines CT-Geräts und dadurch entstehende Kosten einsparen lassen. Ein weiterer Vorteil eines handelsüblichen CT-Geräts liegt darin, dass die Computertomographievorrichtung nicht auf ein bestimmtes CT-Gerät festgelegt ist, sondern mit einer Vielzahl handelsüblicher CT-Geräte, und insbesondere herstellerunabhängig, realisiert werden kann.

Die Erfindung beruht auf der Überlegung, dass ein handelsübliches CT-Gerät an sich nicht geeignet ist, ein Körperteil eines auf einer Standfläche stationär stehenden Großtieres zu untersuchen, da das zu untersuchende Körperteil des Großtieres für die Untersuchung auf dem CT-Tisch durch Gantry fahren muss. Ein Großtier müsste dann seine stationäre Position auf der Standfläche aufgeben, um der Bewegung des einfahrenden CT-Tisches zu folgen. Dadurch würde eine CT-Untersuchung eines Körperteils deutlich erschwert und das Großtier würde zusätzlichem Stress ausgesetzt werden.

Jedoch haben die Erfinder erkannt, dass dieser Nachteil aufgelöst werden kann, wenn die Computertomographievorrichtung eine Plattform und eine mit der Plattform verbundene Verschiebevorrichtung umfasst. Erfindungsgemäß ist das CT-Gerät deshalb auf der Plattform montiert. Die Plattform dient also als Stellfläche für das CT-Gerät und ist ausgebildet ein CT-Gerät zu tragen.

Die Verschiebevorrichtung ist ausgebildet, die Plattform relativ zu der Standfläche eines Großtieres horizontal zu verschieben. Die Standfläche des Großtieres liegt außerhalb der Plattform, sodass sich ein stationär stehendes Großtier selbst nicht bewegt, wenn sich die Plattform mit dem CT-Gerät relativ zu der Standfläche horizontal verschiebt. Während einer CT-Untersuchung liegt jedoch das zu untersuchende Körperteil des Großtieres auf dem CT-Tisch auf und würde mit dem CT-Tisch in die Gantry einfahren. Um ein horizontales Verschieben des zu untersuchenden Körperteils während einer CT-Untersuchung relativ zu der Standfläche, also auch relativ zu dem übrigen Großtier, zu verhindern, ist erfindungsgemäß vorgesehen, dass die Computertomographievorrichtung wenigstens einen Betriebszustand aufweist, in dem die Plattform derart horizontal verschoben wird, dass der CT-Tisch während einer Relativbewegung zur Gantry relativ zu der Standfläche stationär bleibt.

In diesem Betriebszustand der Computertomographievorrichtung steht der CT-Tisch für einen außenstehenden Beobachter still. Für einen außenstehenden Beobachter ergibt sich also ein Bild, als wenn die Gantry, die an sich unbeweglich auf der Plattform montiert ist, über den CT-Tisch und insbesondere über ein zu untersuchendes Körperteil eines Großtieres hinwegfahren würde. In diesem Betriebszustand bewegen sich für einen außenstehenden Beobachter gerade diejenigen Elemente des CT-Geräts, die sich bei einer handelsüblichen Verwendung ohne Plattform und Verschiebevorrichtung nicht bewegen würden. Vorteilhafterweise steht in diesem Betriebszustand also der CT-Tisch für einen außenstehenden Beobachter still, während sich die Gantry über den CT-Tisch und ein zu untersuchender Körperteil eines Großtieres hinwegzubewegen scheint. Durch die Computertomographievorrichtung muss ein stationär stehendes Großtier während einer CT-Untersuchung also nicht seine Position auf der Standfläche aufgeben und bleibt somit unbewegt in Bezug auf das CT-Gerät. Alle für die CT-Untersuchung erforderlichen Bewegungen führt die Computertomographievorrichtung aus.

Ein Großtier steht während einer CT-Untersuchung also unbeweglich auf festem Grund. Vorteilhafterweise bewegt sich ein Großtier dann während einer CT-Untersuchung nicht und insbesondere auch nicht das zu untersuchende Körperteil des Großtieres. Eine CT-Untersuchung kann also besonders zuverlässig an einem stationär stehenden Großtier durchgeführt werden, ohne dass das Großtier besonders narkotisiert werden muss und ohne, dass das Großtier zusätzlichem Stress ausgesetzt wird.

Im Folgenden werden bevorzugte Weiterbildungen der erfindungsgemäßen Computertomographievorrichtung beschrieben.

CT-Tisch und Plattform werden während einer CT-Untersuchung mit vom Betrag her gleicher Geschwindigkeit in jeweils zueinander entgegengesetzte Richtungen verschoben. Dadurch wird die Bewegung des in die Gantry einfahrenden CT-Tisches durch die entgegengesetzt gerichtete Bewegung der Plattform für einen außenstehenden Beobachter kompensiert. Für einen außenstehenden Beobachter steht dann der CT-Tisch still während sich die Gantry über den CT-Tisch hinwegzubewegen scheint. In anderen Worten bewegen sich insbesondere der CT-Tisch und die Plattform derart, dass die Bewegung des CT-Tisches relativ zur Gantry und zur Plattform durch eine Relativbewegung der Plattform zu Standfläche in die dazu entgegengesetzte Richtung, für einen außenstehenden Beobachter und insbesondere für ein auf der Standfläche befindliches Großtier kompensiert wird. Dadurch steht der CT-Tisch für einen außenstehenden Beobachter still, während die Gantry, die an sich unbeweglich auf der Plattform montiert ist, für den außenstehenden Beobachter über den CT-Tisch und insbesondere über ein zu untersuchendes Körperteil eines Großtieres hinwegzufahren scheint.

In einer besonders bevorzugten Weiterbildung weist die Computertomographievorrichtung zusätzlich eine Hebevorrichtung auf. Die Hebevorrichtung ist ausgebildet, die Plattform relativ zu der Standfläche eines Großtieres anzuheben oder abzusenken. Das auf der Plattform montierte CT-Gerät kann mittels der Hebevorrichtung und der Verschiebevorrichtung also relativ zu der Standfläche sowohl vertikal angehoben und abgesenkt als auch horizontal verschoben werden.

Durch eine Hebevorrichtung können vorteilhafterweise mehrere Körperteile eines Großtieres in unterschiedlichen Höhen nacheinander untersucht werden, ohne dass das Großtier seine Position auf der Standfläche wesentlich ändern muss. Zum Beispiel können nacheinander der Kopf, der Hals und die Vorderbeine eines Großtieres untersucht werden. Vorzugsweise ist die Hebevorrichtung ausgebildet, das CT-Gerät in einem Höhenbereich von ein bis zwei Metern anzuheben und abzusenken, sodass beispielsweise die Schulterhöhe eines Pferdes von dem Hubbereich des CT-Geräts umfasst ist. Vorteilhafterweise kann ein Großtier unbeweglich auf festem Grund vor der Computertomographievorrichtung stehen. Besonders vorteilhaft kann also auf eine Hebebühne zum Anheben eines Großtiers auf eine für eine CT-Untersuchung notwendige Höhe verzichtet werden.

Alle nötigen Bewegungen zum Durchführen einer CT-Untersuchung eines oder nacheinander mehrerer Körperteile eines Großtieres werden allein von der Computertomographievorrichtung ausgeführt.

Bevorzugt umfasst die Verschiebevorrichtung ein Schienensystem, welches auf einer Bodenplatte angeordnet ist. Vorzugsweise ist die Plattform mit dem montierten CT-Gerät auf dem Schienensystem angeordnet, sodass die Bodenplatte und die Plattform durch das Schienensystem miteinander verbunden und relativ zueinander beweglich sind. Die Bodenplatte hat vorzugsweise dieselbe Länge und Breite wir die Plattform. Der benötigte Platz zum Installieren der Computertomographievorrichtung erhöht sich also nicht durch die Bodenplatte. Die Bodenplatte kann auch nicht vollflächig, sondern als Rahmen ausgebildet sein. Die Bodenplatte ist ausgebildet, das Gewicht der Plattform und des darauf montierten CT-Geräts zu tragen. Durch das Schienensystem können die Bodenplatte und die Plattform vergleichsweise reibungsarm in eine definierte Richtung zueinander verschoben werden.

Bevorzugt umfasst die Verschiebevorrichtung einen Verschiebemotor, der ausgebildet ist, einen Verschiebeantrieb zum Verschieben der Plattform relativ zu der Standfläche eines Großtieres bereitzustellen. Vorteilhafterweise kann die Verschiebegeschwindigkeit der Plattform bis zu 14 cm pro Sekunde betragen. Der Verschiebemotor kann also vergleichsweise leistungsstark sein. Der Verschiebemotor ist vorzugsweise ein Elektromotor. Der Verschiebemotor ist insbesondere ausgebildet eine Leistung bereitzustellen, die ausreicht, die über ein Schienensystem miteinander verbundene Plattform und Bodenplatte gegeneinander zu verschieben.

In einer Weiterbildung umfasst die Verschiebevorrichtung eine Gewindespindel und ein auf der Gewindespindel verschiebbar gelagertes Gegengewinde. Bevorzugt ist der Verschiebemotor zum Antreiben der Gewindespindel beispielsweise über einen Riemen mit der Gewindespindel verbunden, und das Gegengewinde an der Plattform angeordnet, sodass im Betrieb des Verschiebemotors eine Rotation der Gewindespindel in eine translatorische Verschiebung der Plattform relativ zu der Standfläche umgewandelt wird. Durch die Gewindespindel und dem darauf verschiebbar, aber nicht drehbar gelagerten Gegengewindes kann eine Drehbewegung des Verschiebemotors, der die Gewindespindel antreibt, in eine relative Verschiebebewegung des Gegengewindes und somit der Plattform gegenüber der Gewindespindel umgesetzt werden. Es handelt sich hierbei also um einen Spindelantrieb.

Vorzugsweise umfasst das CT-Gerät einen CT-Motor zum motorbetriebenen Einfahren des CT-Tisches in die Gantry.

In einer Weiterbildung umfasst das CT-Gerät einen CT-Antrieb, insbesondere mit einem CT-Motor, zum motorbetriebenen Einfahren des CT-Tisches in die Gantry und die Verschiebevorrichtung einen Verschiebemotor umfasst. Bevorzugt sind dann der Verschiebeantrieb und der CT-Antrieb durch eine Kopplung mechanisch miteinander gekoppelt.

In einer Weiterbildung ist die Kopplung derart ausgebildet, dass ein CT-Antrieb direkt durch eine mechanische Kopplung abgegriffen und an den Verschiebeantrieb übertragen wird. Eine mechanische Kopplung kann beispielsweise durch eine Spindel realisiert sein, über die der CT-Antrieb und der Verschiebeantrieb miteinander verbunden sind.

In einer Weiterbildung sind der Verschiebeantrieb und der CT-Antrieb derart miteinander gekoppelt, dass sich der CT-Tisch und die Plattform im Betrieb mit vom Betrag her gleicher Geschwindigkeit in jeweils zueinander entgegengesetzte Richtungen verschieben. Vorzugsweise fährt der CT-Tisch in die Öffnung der Gantry ein während die Plattform in die entgegengesetzte Richtung verschoben wird. Beide Bewegungen werden also vorzugsweise zeitgleich ausgeführt. Dadurch, dass sich der CT-Tisch und die Plattform während einer CT-Untersuchung mit vom Betrag her gleicher Geschwindigkeit in zueinander entgegengesetzte Richtung bewegen, wird die Bewegung des CT-Tisches für einen außenstehenden Betrachter durch die Bewegung der Plattform kompensiert, sodass der CT-Tisch für einen außenstehenden Betrachter während einer CT-Untersuchung ortsfest ist.

In einer Weiterbildung ist die Kopplung des CT-Antriebs und des Verschiebeantriebs derart gestaltet, dass ein Startsignal am CT-Motor abgegriffen und an den Verschiebemotor übertragen wird. Die Plattform wird dann vorzugsweise durch den Verschiebemotor aufgrund des empfangenen Startsignals in eine Richtung verschoben, die der Richtung in die der CT-Tisch in die Öffnung der ringförmigen Gantry einfährt entgegengesetzt ist.

Beispielweise können eine Anzahl unterschiedlicher Geschwindigkeiten vorzugsweise in ein Steuergerät des Verschiebemotors einprogrammiert sein. Für eine CT-Untersuchung kann dann ein CT-Protokoll ausgewählt werden, in welchem der CT-Tisch entsprechend einer der einprogrammierten Geschwindigkeiten des Verschiebemotors mit dieser Geschwindigkeit in die Gantry eingefahren wird. Das Startsignal des CT-Motors kann dann abgegriffen und an den Verschiebemotor übertragen werden, sodass sich der CT-Tisch und die Plattform im Wesentlichen zeitgleich mit vom Betrag her gleicher Geschwindigkeit in zueinander entgegengesetzte Richtungen bewegen. Dadurch steht der CT-Tisch für einen außenstehenden Beobachter still, während die Gantry für den außenstehenden Beobachter über den CT-Tisch hinweg fährt.

In einer Weiterbildung ist die Kopplung des CT-Antriebs und des Verschiebeantriebs derart gestaltet, dass die Geschwindigkeit und/oder die Position des CT-Tisches und/oder der Plattform durch einen entsprechenden Geschwindigkeits- oder Positionssensor gemessen wird. Ein Geschwindigkeitssignal und/oder Positionssignal, welche entsprechend eine gemessene Geschwindigkeit oder Position repräsentieren, kann dann an den CT-Motor und/oder den Verschiebemotor als Stellgröße übertragen und verwendet werden, den CT-Motor und/oder den Verschiebemotor zu regeln. Geschwindigkeit und/oder Position stellen dann Stellgrößen dar, mit denen der CT-Motor und/oder der Verschiebemotor, beispielsweise die Drehzahl als Regelgröße, gezielt beeinflusst werden kann. Insbesondere werden die Stellgrößen verwendet den CT-Motor und/oder den Verschiebemotor derart zu regeln, dass sich CT-Tisch und Plattform mit vom Betrag her gleicher Geschwindigkeit in zueinander entgegensetzte Richtungen bewegen. Auch dadurch kann eine Synchronisation der Bewegungen des CT-Tisches und der Plattform derart erreicht werden, dass der CT-Tisch für einen außenstehenden Beobachter stillsteht, während die Gantry über den CT-Tisch hinwegzufahren scheint.

In einer Weiterbildung, in der die Plattform mit dem CT-Gerät auf einer Bodenplatte angeordnet ist, umfasst die Hebevorrichtung eine Mehrzahl Gewindespindeln. Die Gewindespindeln der Hebevorrichtung sind vorzugsweise in Bezug auf die Bodenplatte senkrecht in Richtung der Plattform seitlich an der Bodenplatte angeordnet und jeweils über ein verschiebbar gelagertes Gegengewinde mit der Bodenplatte verbunden. An der Bodenplatte sind also bevorzugt Gegengewinde angebracht, die jeweils verschiebbar auf einer Gewindespindel gelagert sind. Wenn die Gewindespindeln angetrieben werden, kann so die Bodenplatte und die auf der Bodenplatte angeordnete Plattform mit dem CT-Gerät angehobenen oder abgesenkt werden.

Vorzugsweise sind vier Säulen vorgesehen, in denen jeweils eine Gewindespindel angeordnet ist. Die vier Säulen sind bevorzugt seitlich an der Bodenplatte angeordnet, sodass die Gewindespindeln in den Säulen jeweils in Bezug auf die Bodenplatte senkrecht in Richtung der Plattform verlaufen. Die Säulen tragen zur Stabilität der Hebevorrichtung bei und schützen die Gewindespindeln der Hebevorrichtung vor äußeren Einflüssen, beispielsweise Verschmutzung.

In einer Weiterbildung umfasst die Hebevorrichtung wenigstens einen Hebemotor, der zum Antreiben wenigstens einer der Mehrzahl der Gewindespindeln der Hebevorrichtung mit einer der Gewindespindeln verbunden und ausgebildet ist, einen Hebeantrieb zum Anheben oder Absenken der Plattform relativ zu der Standfläche eines Großtieres bereitzustellen. Der Hebemotor ist vorzugsweise ausgebildet, eine Leistung bereitzustellen, die ausreicht eine Gewindespindel anzutreiben und somit die Bodenplatte und die darauf angeordnete Plattform mit CT-Gerät anzuheben oder abzusenken. Auch der Hebemotor kann über Riemen mit einer oder mehreren Gewindespindeln verbunden sein. Der Hebemotor ist vorzugsweise ein Elektromotor.

Vorzugsweise sind wenigstens zwei der Gegengewinde der Hebevorrichtung durch eine Synchronisationsvorrichtung mechanisch miteinander verbunden. Eine Synchronisationsvorrichtung kann beispielsweise durch ein Verbindungselement realisiert sein, welches zwei Gegengewinde mechanisch miteinander koppelt. Ein Verbindungselement kann beispielsweise eine Metallstange oder ein Betonelement sein. Wenn also eine Gewindespindel angetrieben wird und sich das darauf verschiebbar gelagerte Gegenwinde entlang der Gewindespindel verschiebt, verschiebt sich ein über eine Synchronisationsvorrichtung mit dem Gegengewinde verbundenes weiteres Gegengewinde synchron mit dem Gegengewinde der angetriebenen Gewindespindel. Es kann auch vorteilhaft sein, mehr als zwei Gegengewinde über eine Synchronisationsvorrichtung miteinander mechanisch zu verbinden.

In einer Weiterbildung, in der die Plattform mit dem CT-Gerät auf einer Bodenplatte angeordnet ist, umfasst die Hebevorrichtung bevorzugt wenigstens einen hydraulischen Druckzylinder. Der Druckzylinder ist vorzugsweise derart in Bezug auf die Bodenplatte angeordnet, dass die Bodenplatte durch Druckbeaufschlagung des Druckzylinders relativ zu der Standfläche angehoben oder abgesenkt werden kann.

In einer bevorzugten Weiterbildung weist die Computertomographievorrichtung einen Untersuchungsstand auf. Der Untersuchungsstand umfasst vorzugsweise eine Haltevorrichtung und eine von der Haltevorrichtung gehaltene Ablage auf der ein Körperteil eines Großtieres abgelegt werden kann. Der Untersuchungstand ist bevorzugt derart angeordnet, dass sich wenigstens die Ablage während eines Verschiebens der Plattform durch die Verschiebevorrichtung nicht mitbewegt wird. Vorzugsweise ist die Ablage derart ausgebildet, dass sie in einer Höhe in Bezug auf das CT-Gerät verstellt werden kann. Es ist denkbar, dass die Ablage derart an der Haltevorrichtung angeordnet ist, dass die Ablage von der Haltevorrichtung abgenommen und in einer unterschiedlichen Höhe wieder an der Haltevorrichtung angebracht werden kann. Beispielsweise kann die Ablage durch eine Schraubverbindung an der Haltevorrichtung angebracht sein und die Haltevorrichtung entlang der Höhe Löcher zum Befestigen der Ablage durch die Schraubverbindung aufweisen. Die Höhe der Ablage in Bezug auf ein zu untersuchendes Großtier kann also vorteilhafterweise entsprechend der Höhe des Großtieres an das CT-Gerät angepasst werden. Vorzugsweise ist die Ablage derart angeordnet und auf eine Höhe eingestellt, dass die Ablage durch ein Verschieben der Plattform mit dem CT-Gerät in die Öffnung der ringförmigen Gantry eingefahren werden kann, insbesondere soweit, dass die Ablage durch die Öffnung der ringförmigen Gantry hindurch ragt. Die Gantry kann dann über die Ablage hinweg fahren, sodass sich die Ablage während einer CT-Untersuchung wenigstens mit einem Teilbereich innerhalb der Öffnung der ringförmigen Gantry befindet.

Vorzugsweise ist Ablage und insbesondere die Teilbereiche der Ablage, die sich während einer CT-Untersuchung potentiell innerhalb der Gantry befinden können, aus einem Material gebildet, welches transparent für Röntgenstrahlen ist. Auch zum Fixieren eines zu untersuchenden Körperteils auf dem CT-Tisch oder auf der Ablage wird bevorzugt ein Fixierelement, beispielsweise ein Gurt, aus einem röntgentransparenten Material verwendet.

Wenn eine Computertomographievorrichtung eine Bodenplatte umfasst, auf der die Plattform mit dem montierten CT-Gerät angeordnet ist, ist die Haltevorrichtung vorzugsweise an der Bodenplatte angebracht, sodass sich die Haltevorrichtung und die Ablage gemeinsam mit der Bodenplatte bewegen, wenn diese durch eine Hebevorrichtung relativ zu der Standfläche angehoben oder abgesenkt wird.

Dadurch, dass die Haltevorrichtung an der Bodenplatte angebracht ist, ist die Haltevorrichtung mit der Ablage zusammen mit der Bodenplatte höhenverstellbar. Wenn also die Bodenplatte mit einer Hebevorrichtung angehoben oder abgesenkt wird, wird die Haltevorrichtung mit der Ablage entsprechend zusammen mit der Bodenplatte angehoben oder abgesenkt. Die Ablage kann so an die Höhe eines zu untersuchenden Großtieres und insbesondere eines zu untersuchendes Körperteils, beispielsweise an die Höhe eines Pferdekopfes oder Halses, angepasst werden.

Bevorzugt ist die Haltevorrichtung derart ausgebildet, dass sie einen Schutz des CT-Geräts vor einem Einwirken eines zu untersuchenden Großtieres gewährleistet. Beispielsweise kann ein verstellbarer Gurt so an der Haltevorrichtung angebracht sein, dass ein Weg nach vorne in Richtung des CT-Geräts für ein Großtier versperrt ist, um so das CT-Gerät beispielsweise vor einem Tritt zu schützen.

Vorzugsweise ist die Haltevorrichtung derart an der Bodenplatte angeordnet, dass sie leicht abnehmbar ist, beispielsweise durch ein Lösen einer Anzahl von Schrauben oder anderer Befestigungsmittel. Vorteilhafterweise kann dann eine Haltevorrichtung für eine CT-Untersuchung eines Großtieres in Vollnarkose abgenommen und ein OP-Tisch unmittelbar an die Gantry angrenzend positioniert werden.

Da der CT-Tisch während einer CT-Untersuchung relativ zu der Standfläche eines Großtieres stationär bleibt, kann die Ablage auch auf der dem CT-Tisch zugewandten Seite der Ablage mit dem CT-Tisch verbunden sein. Dadurch kann die Stabilität der Ablage erhöht werden. In einer Weiterbildung weist eine Computertomographievorrichtung entsprechend eine Haltevorrichtung mit einer Ablage auf, wobei die Ablage an der dem CT-Tisch zugewandten Seite mit dem CT-Tisch verbunden ist. Durch ein Verschieben der Plattform kann die Ablage dann in die Öffnung der ringförmigen Gantry eingefahren werden.

In einer Weiterbildung ist die Gantry eines handelsüblichen CT-Geräts in Bezug auf den CT-Tisch um 180° gedreht. Vorteilhafterweise befinden sich dann die exzentrisch in der Gantry angeordnete CT-Röhre und der Detektor näher an dem zu untersuchenden Großtier, da sich das Großtier für eine CT-Untersuchung auf der dem CT-Tisch gegenüberliegenden Seite der Gantry befindet und somit auf derjenigen Seite, auf der sich ursprünglich kein zu untersuchender Patient während einer CT-Untersuchung befindet.

In einer Weiterbildung ist der CT-Tisch ausgekoppelt, d.h. der CT-Motor läuft frei. Vorteilhafterweise kann der CT-Tisch dann frei bewegt und auch als Auflagefläche für ein Körperteil eines Großtieres in beliebiger Stellung fixiert werden.

In einer Weiterbildung ist die Ablage auf der dem CT-Tisch zugewandten Seite mit dem CT-Tisch mechanisch verbunden, und der CT-Tisch ausgekoppelt und dadurch in Bezug auf das übrige CT-Gerät frei beweglich, sodass der mit der Ablage verbundene CT-Tisch, wenn das auf der Plattform montierte CT-Gerät zusammen mit der Plattform durch die Verschiebevorrichtung verschoben wird, durch die mechanische Verbindung mit der Ablage relativ zu der Standfläche während einer Relativbewegung zur Gantry stationär bleibt. Wenn also die Plattform durch die Verschiebevorrichtung verschoben wird, bewegt sich das gesamte auf der Plattform montierte CT-Gerät mit der Plattform mit. Die Gantry scheint sich also relativ zu der Standfläche des Großtiers zu verschieben. Der CT-Tisch ist mit der Ablage verbunden, wobei die Ablage so angeordnet ist, dass sie sich nicht mit der Plattform mitbewegt, wenn diese verschoben wird. Die Ablage hält also den CT-Tisch in einer festen Position. Dies ist möglich, da der CT-Tisch ausgekoppelt und dadurch in Bezug auf das übrige CT-Gerät frei beweglich ist. Für einen außenstehenden Beobachter sieht es dann so aus, dass während eines Verschiebens der Plattform der CT-Tisch stillzustehen scheint und die Gantry über den CT-Tisch hinwegzufahren scheint.

Vorzugsweise weist die Gantry einen Laser auf, der in der Gantry derart angeordnet und ausgebildet ist, während einer CT-Untersuchung durch emittiertes Laserlicht das momentan untersuchte Gebiet sichtbar zu machen.

In einer Weiterbildung umfasst die Computertomographievorrichtung Strahlenschutzvorrichtungen, insbesondere Bleimäntel, die jeweils vor und hinter der Gantry angebracht sind. Vorteilhafterweise kann dann Untersuchungspersonal während einer CT-Untersuchung eines Großtieres mitwirken und beispielweise neben einem Pferd ein Pferdebein als auch vor einem Pferd dessen Pferdekopf fixieren ohne einer gesundheitsschädlichen Strahlendosis ausgesetzt zu sein.

Vorher beschriebene Computertomographievorrichtungen können auch innerhalb einer Computertomographie-Untersuchungsanordnung verwendet werden, wobei die Computertomographie-Untersuchungsanordnung eine Computertomographievorrichtung, eine Standfläche für ein Großtier und eine Bodengrube umfasst. Die Computertomographievorrichtung ist in der Bodengrube angeordnet, sodass sich wenigstens ein Teil der Computertomographievorrichtung auf einem im Vergleich zu der Standfläche niedrigeren Niveau befindet. Die Standfläche befindet sich außerhalb der Bodengrube an diejenige Seitenwand der Bodengrube angrenzend, die der Gantry der Computertomographievorrichtung am nächsten ist.

Vorzugsweise ist die Bodengrube so tief in Bezug auf die Standfläche gestaltet, dass sich der CT-Tisch des CT-Geräts, wenn das CT-Gerät durch eine Hebevorrichtung vollständig abgesenkt ist, auf derselben Höhe wie die Standfläche befindet. In diesem Zustand ist es vorteilhafterweise möglich, dass ein Kopf eines Pferdes oberhalb der Gantry positioniert werden kann. Ein Vorderbein des Pferdes kann dann schräg nach vorne auf eine Ablage eines Untersuchungsstandes oder den CT-Tisch gestellt werden, sodass die Gantry während einer CT-Untersuchung relativ zu der Standfläche des Pferdes über das Vorderbein hinwegzufahren scheint. Ein Hinterbein eines Pferdes kann entsprechend von dem Pferd aus schräg nach hinten auf eine Ablage oder den CT-Tisch gestellt werden. Dadurch, dass die Computertomographievorrichtungen vorzugsweise zu 50% in die Bodengrube eingelassen ist, sind besonders vorteilhaft CT-Untersuchungen an den Gliedmaßen eines stehenden Großtieres möglich.

Durch die Hebevorrichtung kann ein CT-Gerät dann vorzugsweise bis zu zwei Meter angehoben werden, sodass im angehobenen Zustand dann auch eine CT-Untersuchung des Kopfes und des Halses eines Großtieres möglich ist. Mit der Computertomographie-Untersuchungsanordnung können also besonders vorteilhaft sowohl die Gliedmaßen als auch der Kopf oder der Hals eines stationär stehenden Großtieres untersucht werden. Insbesondere ermöglicht die Computertomographie-Untersuchungsanordnung, dass eine solche Untersuchung besonders vorteilhaft mit einem handelsüblichen CT-Gerät durchgeführt werden kann.

Wenn sich die Standfläche eines Großtieres auf einem Podest befindet, kann ein Effekt erzielt werden, der dem Anordnen einer Computertomographievorrichtung in einer Bodengrube ähnelt. Das Podest ist dann derart in Bezug auf die Computertomographievorrichtung angeordnet, dass das Pferd an einer identischen Position allerdings erhöht steht. Durch das Podest befindet sich die Standfläche vorzugsweise auf Höhe das CT-Tisches, wenn das CT-Gerät durch eine Hebevorrichtung vollständig abgesenkt ist. In dieser Anordnung lassen sich dann besonders vorteilhaft die Gliedmaßen eines Großtieres mit der Computertomographievorrichtung untersuchen. Durch eine Hebevorrichtung kann das CT-Gerät dann in einem Hubbereich von bis zu zwei Metern angehoben werden, sodass auch der Kopf oder Hals des auf dem Podest stationär stehenden Großtieres vergleichsweise leicht mit der Computertomographievorrichtung untersucht werden können.

Hinsichtlich des Verfahrens wird die eingangs genannte Aufgabe der Erfindung gelöst durch ein Verfahren zum Untersuchen eines Körperteils eines Großtieres mit einer Computertomographievorrichtung. Die Computertomographievorrichtung weist ein Computertomographie (CT)-Gerät und eine Plattform, auf der der CT-Gerät montiert ist, auf. Das CT-Gerät umfasst eine ringförmigen Gantry und einen CT-Tisch zur Aufnahme eines zu untersuchenden Körperteils eines Großtieres. Der CT-Tisch und die Gantry sind zueinander beweglich miteinander verbunden, sodass der CT-Tisch in eine Öffnung der ringförmigen Gantry eingefahren werden kann.

Das erfindungsgemäße Verfahren weist die Schritte auf:
- Positionieren eines Großtieres auf einer Standfläche,
- Auflegen eines zu untersuchenden Körperteils auf den CT-Tisch oder eine Ablage des CT-Geräts,
- Verschieben der Plattform mit dem CT-Gerät relativ zu der Standfläche,
wobei das Verschieben der Plattform in einem Betriebszustand derart gestaltet ist, dass der CT-Tisch oder die Ablage während einer Relativbewegung zur Gantry relativ zu der Standfläche stationär bleiben, und wobei in dem Betriebszustand der CT-Tisch in die Öffnung der ringförmigen Gantry in eine erste Richtung einfährt, und die Plattform in eine zweite Richtung, die der ersten Richtung entgegengesetzt ist verschoben wird, wobei das Einfahren des CT-Tisches und das Verschieben der Plattform jeweils mit einer vom Betrag her gleichen Geschwindigkeit durchgeführt werden, sodass der CT-Tisch relativ zu der Standfläche stationär bleibt.

Dabei werden also das Einfahren des CT-Tisches und das Verschieben der Plattform in zueinander entgegengesetzte Richtung mit jeweils einer vom Betrag her gleichen Geschwindigkeit durchgeführt, sodass der CT-Tisch relativ zu einer Standfläche eines Großtieres stationär bleibt.

Bevorzugt umfasst das Verfahren weiterhin ein
- Anheben oder Absenken der Plattform relativ zu einer Standfläche eines Großtieres.

Die Erfindung soll nun anhand eines Ausführungsbeispiels in Bezug auf die Figuren näher erläutert werden. Von den Figuren zeigt:
- Fig. 1:: eine Computertomographievorrichtung zum Untersuchen eines Körperteils eines Großtieres;
- Fig. 2:: eine Computertomographie-Untersuchungsanordnung aufweisend eine in einer Bo-dengrube angeordnete Computertomographievorrichtung in einer Seitenansicht;
- Fig. 3:: eine Computertomographievorrichtung mit Haltevorrichtung und Ablage;
- Fig. 4:: eine Computertomographievorrichtung zum Untersuchen eines Körperteils eines Großtieres.

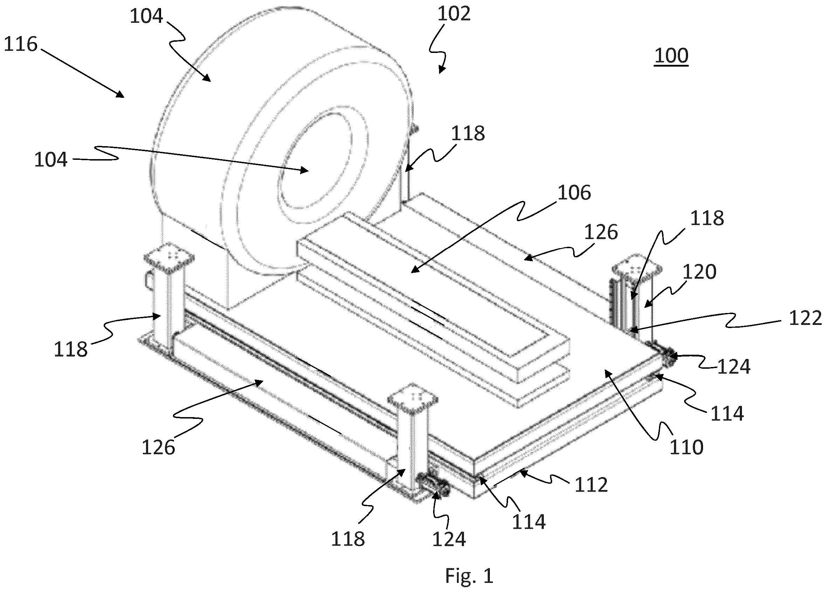

In Figur 1 ist eine Computertomographievorrichtung 100 zum Untersuchen eines Körperteils eines Großtieres gezeigt.

Die Computertomographievorrichtung 100 weist ein CT-Gerät 102 auf, welches eine ringförmige Gantry 104 und einen CT-Tisch 106 umfasst. Die Gantry 104 ist als Ringtunnel ausgebildet, in dem eine CT-Röhre und gegenüber von der CT-Röhre ein Detektor (beide nicht gezeigt) angeordnet sind. Mittig weist die ringförmige Gantry 104 eine Öffnung 108 auf. Der CT-Tisch 106 und die Gantry 104 sind zueinander beweglich miteinander verbunden, sodass der CT-Tisch 106 in die Öffnung 108 der ringförmigen Gantry 104 eingefahren werden kann. Ein wie in Figur 1 gezeigtes CT-Gerät 102 ist grundsätzlich im freien Handel verfügbar.

Die Computertomographievorrichtung 100 weist weiterhin eine Plattform 110 auf, auf der das CT-Gerät 102 montiert ist. Die Plattform 110 ist auf einer Bodenplatte 112 angeordnet, wobei sich zwischen der Bodenplatte 112 und der Plattform110 ein Schienensystem 114 befindet. Das Schienensystem 114 ermöglicht, dass die Bodenplatte 112 und die Plattform 110 zueinander vergleichsweise reibungsarm in diejenigen Richtungen hin und her verschoben werden kann, in die auch der CT-Tisch 106 beweglich ist. Das Schienensystem 114 ist ein Element einer Verschiebevorrichtung, welche weiterhin einen Verschiebemotor (nicht gezeigt) und eine von dem Verschiebemotor antreibbare Gewindespindel (nicht gezeigt) aufweist, wobei auf der Gewindespindel ein Gegengewinde (nicht gezeigt) verschiebbar gelagert ist. Das Gegengewinde ist fest mit der Plattform 110 verbunden, sodass im Betrieb des Verschiebemotors eine Rotation der Gewindespindel in eine translatorische Verschiebung der Plattform 110 relativ zu einer Standfläche eines Großtieres umgewandelt wird. Die Standfläche befindet sich hierbei auf derjenigen Seite 116 der Gantry 104, die dem CT-Tisch 106 gegenüberliegt. Die Standfläche, auf der ein Großtier während einer CT-Untersuchung stationär steht, befindet sich also außerhalb der Computertomographievorrichtung 100. Durch die Verschiebevorrichtung kann die Plattform 110 mit dem CT-Gerät 104 also relativ zu einer Standfläche eines Großtieres verschoben werden.

Die Computertomographievorrichtung 100 weist wenigstens einen Betriebszustand auf, in dem die Plattform 110 derart verschoben wird, dass der CT-Tisch 106 während einer Relativbewegung zur Gantry relativ zu der Standfläche stationär bleibt. Beispielsweise können in diesem Betriebszustand insbesondere der CT-Tisch 106 und die Plattform 104 derart bewegen, dass die Bewegung des CT-Tisches 106 durch eine Bewegung der Plattform 110 in die dazu entgegengesetzte Richtung, für einen außenstehenden Beobachter und insbesondere für ein auf der Standfläche befindliches Großtier kompensiert wird. Für einen außenstehenden Betrachter ergibt sich dann ein Bild als wenn die Gantry über den CT-Tisch hinwegfahren würde während der CT-Tisch stillsteht.

In diesem Betriebszustand würde dann der CT-Tisch 106 wie vorgesehen in die Öffnung 108 der Gantry104 einfahren und damit eigentlich auf ein dahinterstehendes Großtier zu. Die Plattform 110 mit dem CT-Gerät 102 bewegt sich jedoch mit einer vom Betrag her gleichen Geschwindigkeit von der Standfläche des Großtieres weg. Dadurch bleibt ein Körperteil, welches auf den CT-Tisch gestellt oder gelegt ist, relativ zu der Standfläche in unbewegter Position, während die Gantry 104 über den Körperteil hinwegzufahren scheint. Für einen außenstehenden Beobachter scheint der der CT-Tisch 106 also in diesem Betriebszustand stillzustehen und die Gantry 104 scheint über den CT-Tisch 106, auf dem ein zu untersuchendes Körperteil eines Großtieres gelegt oder gestellt sein kann, hinwegzufahren.

In der gezeigten Ausführungsform weist das CT-Gerät 102 einen CT-Motor (nicht gezeigt) auf, der ausgebildet den CT-Tisch 106 motorbetrieben zu verfahren. In der gezeigten Ausführungsform sind der CT-Motor und der Verschiebemotor derart gekoppelt, dass ein Startsignal am CT-Motor abgegriffen und an den Verschiebemotor übertragen wird, sobald der CT-Tisch durch CT-Motor angetrieben in Richtung der Öffnung 108 der Gantry 104 fährt. In den Verschiebemotor oder in ein Steuergerät des Verschiebemotors sind eine Anzahl vordefinierter Geschwindigkeiten einprogrammiert und eine der einprogrammierten Geschwindigkeiten für eine CT-Untersuchung ausgewählt. Entsprechend wird für eine CT-Untersuchung ein CT-Protokoll so ausgewählt, dass sich der CT-Tisch während einer CT-Untersuchung mit der ausgewählten Geschwindigkeit des Verschiebemotors bewegt. Der CT-Motor und der Verschiebemotor sind also an sich unabhängig voneinander implementiert. Jedoch werden die Geschwindigkeiten der beiden Motoren über das Startsignal synchronisiert. Die Plattform 110 wird dann durch den Verschiebemotor aufgrund des empfangenen Startsignals mit vom Betrag her gleicher Geschwindigkeit im Wesentlichen zeitgleich in eine Richtung verschoben, die der Richtung in die der CT-Tisch 106 in die Öffnung 108 der ringförmigen Gantry 104 einfährt entgegengesetzt ist. Für einen außenstehenden Beobachter scheint der CT-Tisch106 dann stillzustehen und die Gantry 104 scheint über den CT-Tisch 106 und insbesondere über ein zu untersuchendes Körperteil eines Großtieres hinwegzufahren.

Alternativ zu einer Kopplung durch Abgreifen eines Startsignals, kann die Kopplung des CT-Motors und des Verschiebemotors derart gestaltet sein, dass der CT-Antrieb und der Verschiebeantrieb mechanisch miteinander verbunden sind. In einer nicht gezeigten Ausführungsform sind der CT-Antrieb und der Verschiebeantrieb über eine Spindel miteinander verbunden. Die Bewegung wird dann direkt an dem CT-Tisch abgegriffen und mechanisch über eine Spindel an den Verschiebeantrieb übermittelt werden. Dadurch wird die Bewegung der Plattform direkt an die Bewegung CT-Tisches gekoppelt. Vorteilhafterweise kann so eine Synchronisation der Bewegungen des CT-Tisches und der Plattform weiter verbessert werden. Wird der CT-Tisch beispielsweise händisch oder über die Steuerkonsole bewegt, so bewegt sich die Plattform mit vom Betrag her gleicher Geschwindigkeit und insbesondere in eine Richtung, die der Einfahrrichtung des CT-Tisches in die Gantry entgegengesetzt ist. Dadurch scheint die Gantry für einen außenstehenden Beobachter über den stillstehenden CT-Tisch hinwegzufahren.

In einer weiteren hier nicht gezeigten Ausführungsform sind der CT-Antrieb und der Verschiebeantrieb derart gekoppelt, dass die Geschwindigkeit und/oder die Position des CT-Tisches und/oder der Plattform durch einen entsprechenden Geschwindigkeits- oder Positionssensor gemessen werden. Ein Geschwindigkeitssignal und/oder Positionssignal, welche entsprechend die gemessene Geschwindigkeit oder Position repräsentieren, kann dann an den CT-Motor und/oder den Verschiebemotor übertragen und verwendet werden, den CT-Motor und/oder den Verschiebemotor derart zu regeln, dass sich CT-Tisch und Plattform mit vom Betrag her gleicher Geschwindigkeit in zueinander entgegengesetzte Richtungen bewegen.

Seitlich an der Bodenplatte 112 sind vier Säulen 118 angeordnet in denen jeweils eine Gewindespindel 120 angeordnet ist. Die Gewindespindeln 120 sind Elemente einer Hebevorrichtung und in Bezug auf die Bodenplatte 112 senkrecht in Richtung der Plattform 110 seitlich an der Bodenplatte 112 angeordnet. Jede der Gewindespindeln 120 ist über jeweils ein verschiebbar gelagertes Gegengewinde 122 mit der Bodenplatte 112 verbunden. Die Gegengewinde 122 sind also fest mit der Bodenplatte 112 verbunden und jeweils verschiebbar auf einer Gewindespindel 120 gelagert. Wenn die Gewindespindeln 120 der Hebevorrichtung angetrieben werden, kann so die Bodenplatte 112 und die auf der Bodenplatte 112 angeordnete Plattform 110 mit dem CT-Gerät 102 angehobenen oder abgesenkt werden. Zum Antreiben der Gewindespindeln 120 der Hebevorrichtung sind zwei der Gewindespindeln 120 jeweils mit einem Hebemotor 124 verbunden.

Die Gegengewinde 122 der Hebevorrichtung, die in Säulen 118 auf derselben Längsseite der Bodenplatte angeordnet sind, sind durch eine Synchronisationsvorrichtung 126 mechanisch miteinander verbunden. Die Synchronisationsvorrichtungen 126 sind als durchgehende Verbindungselemente ausgebildet, und koppeln jeweils zwei Gegengewinde einer Längsseite der Bodenplatte mechanisch miteinander. Die Verbindungselemente 126 sind auch mit der Bodenplatte 112 verbunden, sodass die Verbindung der Gegengewinde 122 mit der Bodenplatte 112 zusätzlich unterstützt wird.

Wenn die Gewindespindeln 120 der Hebevorrichtung, die direkt von dem Hebemotor angetrieben werden können, im Motorbetrieb angetrieben werden und sich die darauf verschiebbar gelagerten Gegenwinde 122 jeweils entlang der Gewindespindeln 120 verschieben, verschieben sich die über die Synchronisationsvorrichtungen 126 mit den Gegengewinden 118 verbundenen weiteren Gegengewinde 118 synchron mit den Gegengewinden 118 der angetriebenen Gewindespindeln 120. Dadurch kann die Bodenplatte 112 gleichmäßig angehoben oder abgesenkt werden.

In hier nicht gezeigten Ausführungsformen weist die Hebevorrichtung zusätzlich oder alternativ zu einem Spindelantrieb wenigstens einen hydraulischen Druckzylinder auf. In einer solchen Ausführungsform ist ein Druckzylinder derart in Bezug auf eine Bodenplatte angeordnet ist, dass die Bodenplatte durch Druckbeaufschlagung des Druckzylinders relativ zu einer Standfläche angehoben oder abgesenkt werden kann.

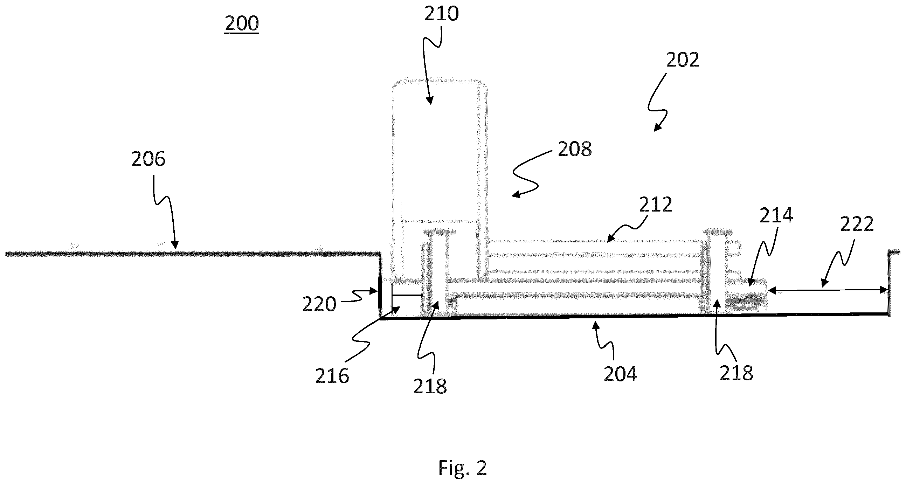

In Figur 2 ist eine Computertomographie-Untersuchungsanordnung 200 aufweisend eine in einer Bodengrube angeordnete Computertomographievorrichtung 202, eine Bodengrube 204 und eine Standfläche für ein Großtier 206 in einer Seitenansicht gezeigt.

Die Computertomographievorrichtung 202 ist analog zu der in Bezug auf Figur 1 beschriebenen Computertomographievorrichtung ausgebildet und umfasst insbesondere ein CT-Gerät 208 mit einer Gantry 210 und einem CT-Tisch 212, der mit einem CT-Motor in eine Öffnung der ringförmigen Gantry 208 eingefahren werden kann. Das CT-Gerät 208 ist auf einer Plattform 214 montiert, die auf einer Bodenplatte 216 angeordnet ist. Die Plattform mit dem CT-Gerät 208 kann durch eine Verschiebevorrichtung (nicht gezeigt) relativ zu der Standfläche 206 in diejenige Richtung hin und her verschoben werden, in die auch der CT-Tisch 212 beweglich ist. Die Bodenplatte 216 kann durch eine Hebevorrichtung 218 angehoben und abgesenkt werden, sodass das auf der Plattform 214 montierte CT-Gerät 208 mittels der Hebevorrichtung 218 und der Verschiebevorrichtung relativ zu der Standfläche 206 sowohl vertikal angehoben und abgesenkt als auch horizontal verschoben werden kann.

Wie aus Figur 2 hervorgeht, befindet sich die Standfläche 206 außerhalb der Bodengrube 204 und zwar angrenzend an diejenige Seitenwand 220 der Bodengrube 206, die der Gantry 210 der Computertomographievorrichtung 202 am nächsten ist. Die Bodengrube 204 weist eine Tiefe auf die so bemessen ist, dass sich der CT-Tisch 212 des CT-Geräts208, wenn das CT-Gerät 202 wie in Figur 2 dargestellt durch die Hebevorrichtung 218 vollständig abgesenkt ist, auf derselben Höhe wie die Standfläche 206 befindet.

In dem gezeigten Ausführungsbeispiel kann der Kopf eines Großtieres oberhalb der Gantry positioniert werden, sodass sich dann der Rumpf des Großtieres unmittelbar vor der Gantry befindet. In diesem vollständig abgesenkten Zustand kann dann beispielsweise ein Vorderbein des Großtieres schräg nach vorne auf den CT-Tisch 212 gestellt werden, sodass die Gantry 210 während einer CT-Untersuchung relativ zu der Standfläche 206 des Großtieres über das Vorderbein hinwegzufahren scheint. Ein Hinterbein eines Großtieres kann entsprechend von dem Großtier aus schräg nach hinten auf den CT-Tisch 212 gestellt werden.

Die Bodengrube 204 weist eine laterale Ausdehnung auf, die es erlaubt, dass die Plattform 214 relativ zu der Bodenplatte 216 durch eine Verschiebevorrichtung verschoben werden kann, ohne an die Seitenwände der Bodengrube 204 zu stoßen. Die Bodengrube 204 weist also insbesondere an den entsprechenden Seiten der Computertomographievorrichtung 202 einen vergleichsweise größeren Abstand 222 zu der Computertomographievorrichtung 202 auf. An den beiden Längsseiten der Computertomographievorrichtung 202, entlang der während einer CT-Untersuchung keine Verschiebung der Plattform214 relativ zu der Bodenplatte 216 stattfindet, können die Seitenwände der Bodengrube 204 vergleichsweise nah an die Computertomographievorrichtung 202 angrenzen.

Durch die Hebevorrichtung 218 kann das CT-Gerät 210 bis zu zwei Meter angehoben werden. In einer hier nicht gezeigten Ausführungsform kann ein CT-Gerät über zwei Meter hinaus, beispielsweise bis zu drei Meter, angehoben werden. Im angehobenen Zustand kann dann beispielsweise eine CT-Untersuchung des Kopfes und des Halses eines Großtieres durchgeführt werden. Mit der gezeigten Computertomographie-Untersuchungsanordnung 200 können also sowohl die Gliedmaßen als auch der Kopf oder der Hals eines stationär stehenden Großtieres untersucht werden. Insbesondere können mit der gezeigten Computertomographie-Untersuchungsanordnung 200 mit demselben handelsüblichen CT-Gerät 202 nacheinander Kopf, Hals und Vorderbeine eines stationär stehenden Großtieres untersucht werden, ohne dass das Großtier seine stationäre Position auf der Standfläche 206 verändern oder aufgeben muss.

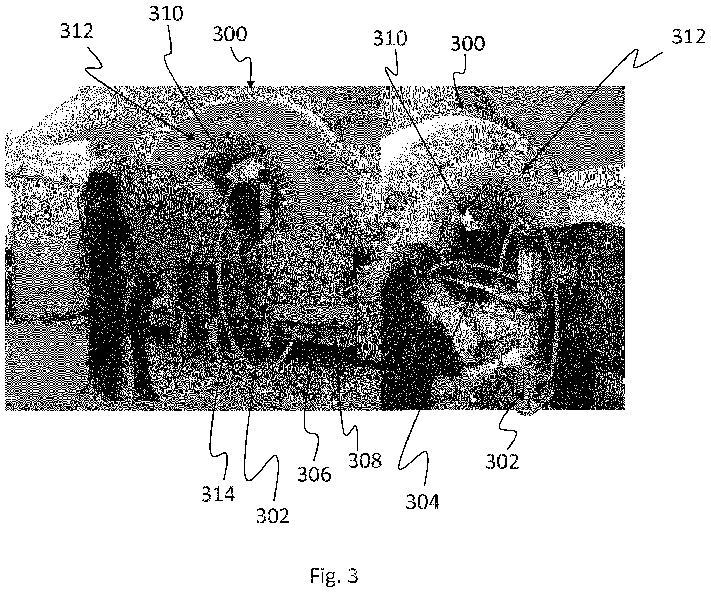

Figur 3 zeigt zwei Ansichten einer Computertomographievorrichtung 300 mit einem Untersuchungsstand, der eine Haltevorrichtung 302 und eine Ablage 304 umfasst.

Die Ablage 304 ist an der Haltevorrichtung 302 angebracht und derart gestaltet, dass ein Körperteil eines Großtieres, hier eines Pferdes, auf der Ablage 304 abgelegt werden kann. Die Haltevorrichtung ist an einer Bodenplatte 306 angebracht, sodass sich Haltevorrichtung 302 und Ablage 304 während eines Verschiebens der Plattform 308 durch eine Verschiebevorrichtung nicht mitbewegen. Wenn allerdings die Bodenplatte 306 durch eine Hebevorrichtung angehoben oder abgesenkt wird, bewegen sich die Haltevorrichtung 302 und die Ablage 304 gemeinsam mit der Bodenplatte 306, sodass die Ablage 304 auf eine bestimmte Höhe in Bezug auf das zu untersuchende Großtier eingestellt werden kann. Die Ablage 304 kann so an die Höhe eines zu untersuchenden Großtieres und insbesondere eines zu untersuchenden Körperteils, beispielsweise an die Höhe eines Pferdekopfes oder Halses, angepasst werden.

Die Ablage 304 ist jedoch in einer Höhe in Bezug auf das CT-Gerät angeordnet, dass die Ablage 304 durch ein Verschieben der Plattform 308 mit dem CT-Gerät in die Öffnung 310 der ringförmigen Gantry 312 eingefahren werden kann, insbesondere soweit, dass die Ablage 304 durch die Öffnung 310 der ringförmigen Gantry 312 hindurch ragt. Während einer CT-Untersuchung scheint die Gantry 312 also für einen außenstehenden Betrachter über die Ablage 304 hinwegzufahren.

An der Computertomographievorrichtung 300 ist auf derjenigen Seite, die der Standfläche eines Großtieres zugewandt ist eine Metall-Konstruktion 314 angebracht. Die Metall-Konstruktion 314 dient als Schutz für das CT-Gerät vor einem Einwirken des Großtieres, beispielsweise einem Tritt, auf das CT-Gerät.

In der gezeigten Ausführungsform einer Computertomographievorrichtung 300 mit einem Untersuchungsstand kann die Ablage auf der dem CT-Tisch zugewandten Seite mit dem CT-Tisch mechanisch verbunden werden. Wenn der CT-Tisch ausgekoppelt und dadurch in Bezug auf das übrige CT-Gerät frei beweglich ist, wird der CT-Tisch nicht mit der Plattform und dem übrigen CT-Gerät verschoben, wenn die Plattform durch die Verschiebevorrichtung verschoben wird. Der CT-Tisch wird dann durch die mechanische Verbindung mit der Ablage für einen außenstehenden Beobachter unbeweglich gehalten. Wenn das CT-Gerät zusammen mit der Plattform durch die Verschiebevorrichtung verschoben wird, scheint der CT-Tisch durch die mechanische Verbindung mit der Ablage relativ zu der Standfläche stationär zu bleiben, während die Gantry über den CT-Tisch relativ zu der Standfläche hinwegzufahren scheint.

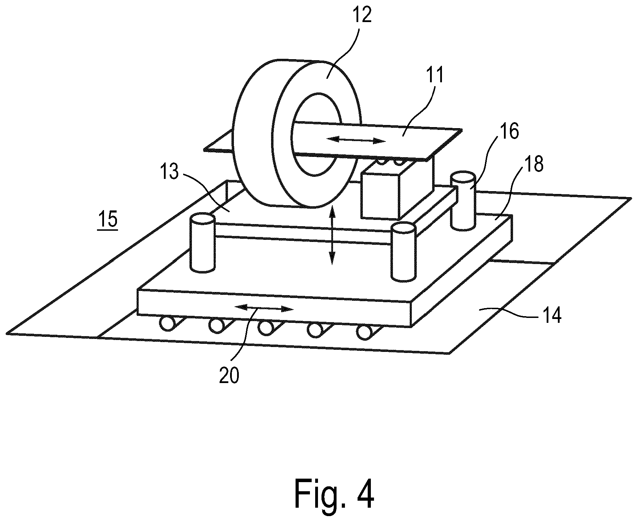

In Figur 4 ist eine Computertomographievorrichtung 15 zum Untersuchen eines Körperteils eines Großtieres gezeigt. Die Computertomographievorrichtung 15 umfasst ein CT-Gerät, welches eine Gantry 12 und einen CT-Tisch 11 umfasst. Das CT-Gerät ist auf einer Bodenplatte 13 angeordnet, die mittels einer Hebevorrichtung 16 vertikal angehoben oder abgesenkt werden kann. Die Gantry 12 ist auf der Bodenplatte 13 unbeweglich angeordnet. Der CT-Tisch 16 ist auf der Bodenplatte 13 horizontal beweglich angeordnet und kann sich relativ zu der Gantry und/oder der Bodenplatte horizontal bewegen. Die Bodenplatte 13 und die Hebevorrichtung 16 sind auf einer Plattform 18 angeordnet. Die Plattform 18 ist auf festem Untergrund 14 beispielsweise in einer Bodengrube angeordnet und kann mittels einer Verschiebevorrichtung 20 horizontal in Bezug auf den Untergrund 14 verschoben werden.

### Bezuqszeichenliste

- 11: CT-Tisch
- 12: Gantry
- 13: Bodenplatte
- 14: Untergrund
- 15: Computertomographievorrichtung
- 16: Hebevorrichtung
- 18: Plattform
- 20: Verschiebevorrichtung
- 100: Computertomographievorrichtung
- 102: CT-Gerät
- 104: Gantry
- 106: CT-Tisch
- 108: Öffnung
- 110: Plattform
- 112: Bodenplatte
- 114: Schienensystem
- 116: Seite der Gantry, die dem CT-Tisch gegenüberliegt
- 118: Säulen der Hebevorrichtung
- 120: Gewindespindeln der Hebevorrichtung
- 122: Gegengewinde der Hebevorrichtung
- 124: Hebemotor
- 126: Synchronisationsvorrichtungen
- 200: Computertomographie-Untersuchungsanordnung
- 202: Computertomographievorrichtung
- 204: Bodengrube
- 206: Standfläche für ein Großtier
- 208: CT-Gerät
- 210: Gantry
- 212: CT-Tisch
- 214: Plattform
- 216: Bodenplatte
- 218: Hebevorrichtung
- 220: Seitenwand
- 222: Abstand zwischen Bodenplatte und Seitenwand
- 300: Computertomographievorrichtung
- 302: Haltevorrichtung
- 304: Ablage
- 306: Bodenplatte
- 308: Plattform
- 310: Öffnung
- 312: Gantry
- 314: Metall-Konstruktion

## Patentansprüche

1. Computertomographievorrichtung (100) zum Untersuchen eines Körperteils eines Großtieres aufweisend:
- ein Computertomographie (CT)-Gerät (102) mit einer ringförmigen Gantry (104) und einen CT-Tisch (106) zur Aufnahme eines zu untersuchenden Körperteils eines Großtieres,
- und eine Plattform (110), auf der das CT-Gerät (102) montiert ist,
wobei die Gantry (104) relativ zu der Plattform unbeweglich ist, und
wobei der CT-Tisch (106) relativ zu der Plattform und zu der Gantry horizontal beweglich mit der Gantry und/oder der Plattform derart verbunden ist, dass der CT-Tisch (106) in eine Öffnung (108) der ringförmigen Gantry (104) eingefahren werden kann, wobei die Computertomographievorrichtung (100) eine Verschiebevorrichtung umfasst, die mit der Plattform (110) verbunden und ausgebildet ist, die Plattform (110) mit dem montierten CT-Gerät (102) relativ zu einer Standfläche eines Großtieres horizontal zu verschieben,
wobei die Standfläche außerhalb der Plattform liegt, **dadurch gekennzeichnet, dass** die Verschiebevorrichtung ausgebildet ist, in wenigstens einem Betriebszustand die Plattform (110) mit dem CT-Gerät (102) derart zu verschieben, dass der CT-Tisch (106) während einer Relativbewegung zur Gantry relativ zu der Standfläche stationär bleibt, indem der CT-Tisch relativ zu der Plattform in die Öffnung der ringförmigen Gantry in eine erste Richtung einfährt, und die Plattform in eine zweite Richtung, die der ersten Richtung entgegengesetzt ist relativ zu der Standfläche verschoben wird, wobei das Einfahren des CT-Tisches und das Verschieben der Plattform jeweils mit einer vom Betrag her gleichen Geschwindigkeit durchgeführt werden, sodass der CT-Tisch relativ zu der Standfläche stationär bleibt.

2. Computertomographievorrichtung (100) nach Anspruch 1, die zusätzlich eine Hebevorrichtung aufweist, wobei die Hebevorrichtung ausgebildet ist, die Plattform (110) relativ zu der Standfläche eines Großtieres vertikal anzuheben oder abzusenken, sodass das auf der Plattform (110) montierte CT-Gerät (102) mittels der Hebevorrichtung und der Verschiebevorrichtung relativ zu der Standfläche sowohl vertikal angehoben und abgesenkt als auch horizontal verschoben werden kann.

3. Computertomographievorrichtung (100) nach Anspruch 1 oder 2, wobei die Verschiebevorrichtung ein Schienensystem (114) umfasst, welches auf einer Bodenplatte (112) angeordnet ist, und wobei die Plattform (110) mit dem montierten CT-Gerät (102) auf dem Schienensystem (114) angeordnet ist, sodass die Bodenplatte (112) und die Plattform (110) durch das Schienensystem (114) relativ zueinander beweglich miteinander verbunden sind.

4. Computertomographievorrichtung (100) nach wenigstens einem der Ansprüche 1 bis 3, wobei die Verschiebevorrichtung einen Verschiebemotor umfasst, der ausgebildet ist, einen Verschiebeantrieb zum Verschieben der Plattform (110) relativ zu der Standfläche eines Großtieres bereitzustellen, und wobei die Verschiebevorrichtung eine Gewindespindel und ein auf der Gewindespindel verschiebbar gelagertes Gegengewinde umfasst, wobei der Verschiebemotor zum Antreiben der Gewindespindel mit der Gewindespindel verbunden ist, und das Gegengewinde an der Plattform (110) angeordnet ist, sodass im Betrieb des Verschiebemotors eine Rotation der Gewindespindel in eine translatorische Verschiebung der Plattform (110) relativ zu der Standfläche umgewandelt wird.

5. Computertomographievorrichtung (100) nach wenigstens einem der Ansprüche 1 bis 4, wobei das CT-Gerät einen CT-Antrieb zum motorbetriebenen Einfahren des CT-Tisches (106) in die Gantry (104) umfasst, und wobei ein Verschiebeantrieb und der CT-Antrieb durch eine Kopplung mechanisch miteinander gekoppelt sind, wobei die Kopplung derart gestaltet ist, dass der CT-Antrieb direkt abgegriffen und an den Verschiebeantrieb übertragen wird.

6. Computertomographievorrichtung (100) nach Anspruch 5, wobei die mechanische Kopplung des CT-Antriebs und des Verschiebeantriebs derart gestaltet ist, dass sich der CT-Tisch und die Plattform (110) im Betrieb mit vom Betrag her gleicher Geschwindigkeit in jeweils zueinander entgegengesetzte Richtungen verschieben.

7. Computertomographievorrichtung (100) nach wenigstens einem der Ansprüche 2 bis 6, wobei die Plattform (110) mit dem CT-Gerät (102) auf einer Bodenplatte (112) angeordnet ist und die Hebevorrichtung eine Mehrzahl Gewindespindeln (120) umfasst, die in Bezug auf die Bodenplatte (112) senkrecht in Richtung der Plattform (110) seitlich an der Bodenplatte (112) angeordnet und jeweils über ein verschiebbar gelagertes Gegengewinde (122) mit der Bodenplatte (112) verbunden sind.

8. Computertomographievorrichtung nach Anspruch 7, wobei die Hebevorrichtung wenigstens einen Hebemotor (124) umfasst, der zum Antreiben wenigstens einer der Mehrzahl der Gewindespindeln (120) der Hebevorrichtung mit einer der Gewindespindeln (120) verbunden und ausgebildet ist, einen Hebeantrieb zum Anheben oder Absenken der Plattform (110) relativ zu der Standfläche eines Großtieres bereitzustellen.

9. Computertomographievorrichtung nach wenigstens einem der Ansprüche 2 bis 8, wobei die Plattform mit dem CT-Gerät auf einer Bodenplatte angeordnet ist und die Hebevorrichtung wenigstens einen hydraulischen Druckzylinder umfasst, der derart in Bezug auf die Bodenplatte angeordnet ist, dass die Bodenplatte durch Druckbeaufschlagung des Druckzylinders relativ zu der Standfläche angehoben oder abgesenkt werden kann.

10. Computertomographievorrichtung (300) nach wenigstens einem der Ansprüche 1 bis 9 die einen Untersuchungsstand aufweist, wobei der Untersuchungsstand eine Haltevorrichtung (302) und eine von der Haltevorrichtung (302) gehaltene Ablage (304) umfasst, wobei der Untersuchungsstand derart angeordnet ist, dass die Ablage (304) während eines Verschiebens der Plattform durch die Verschiebevorrichtung nicht mitbewegt wird.

11. Computertomographievorrichtung (300) nach Anspruch 10 die eine Bodenplatte umfasst, auf der die Plattform mit dem montierten CT-Gerät angeordnet ist, wobei die Haltevorrichtung (302) an der Bodenplatte angebracht ist, sodass sich die Haltevorrichtung (302) und die Ablage (304) gemeinsam mit der Bodenplatte bewegen, wenn diese durch eine Hebevorrichtung relativ zu der Standfläche angehoben oder abgesenkt wird.

12. Computertomographievorrichtung (300) nach Anspruch 10 oder 11, wobei die Ablage (304) auf der dem CT-Tisch zugewandten Seite mit dem CT-Tisch mechanisch verbunden ist, und wobei der CT-Tisch ausgekoppelt und dadurch in Bezug auf das übrige CT-Gerät frei beweglich ist, sodass der mit der Ablage (304) verbundene CT-Tisch, wenn das auf der Plattform montierte CT-Gerät zusammen mit der Plattform durch die Verschiebevorrichtung verschoben wird, durch die mechanische Verbindung mit der Ablage relativ zu der Standfläche während einer Relativbewegung zur Gantry stationär bleibt.

13. Computertomographie-Untersuchungsanordnung (200) aufweisend eine Computertomographievorrichtung (202) nach wenigstens einem der Ansprüche 1 bis 12, eine Standfläche (204) für ein Großtier und eine Bodengrube (206), wobei die Computertomographievorrichtung (202) in der Bodengrube (204) angeordnet ist, sodass sich wenigstens ein Teil der Computertomographievorrichtung (202) auf einem im Vergleich zu der Standfläche (204) niedrigeren Niveau befindet, und wobei sich die Standfläche (204) außerhalb der Bodengrube (206) an diejenige Seitenwand (220) der Bodengrube (206) angrenzend befindet, die der Gantry (210) der Computertomographievorrichtung (202) am nächsten ist.

14. Verfahren zum Untersuchen eines Körperteils eines Großtieres mit einer Computertomographievorrichtung, wobei die Computertomographievorrichtung aufweist:
- ein Computertomographie (CT)-Gerät mit einer ringförmigen Gantry und einem CT-Tisch zur Aufnahme eines zu untersuchenden Körperteils eines Großtieres, und
- eine Plattform, auf der das CT-Gerät montiert ist,
wobei die Gantry (104) relativ zu der Plattform unbeweglich ist,
wobei der CT-Tisch (106) relativ zu der Plattform und zu der Gantry horizontal beweglich mit der Gantry und/oder der Plattform derart verbunden ist,
dass der CT-Tisch (106) in eine Öffnung (108) der ringförmigen Gantry (104) eingefahren werden kann,
wobei das Verfahren die Schritte aufweist:
- Positionieren eines Großtieres auf einer Standfläche,
- Auflegen eines zu untersuchenden Körperteils auf den CT-Tisch,
- Verschieben der Plattform mit dem CT-Gerät relativ zu der Standfläche,
wobei die Verschiebevorrichtung ausgebildet ist, in wenigstens einem Betriebszustand die Plattform (110) mit dem CT-Gerät (102) derart zu verschieben, dass der CT-Tisch (106) während einer Relativbewegung zur Gantry relativ zu der Standfläche stationär bleibt,
wobei in dem Betriebszustand der CT-Tisch relativ zu der Plattform in die Öffnung der ringförmigen Gantry in eine erste Richtung einfährt, und die Plattform in eine zweite Richtung, die der ersten Richtung entgegengesetzt ist relativ zu der Standfläche verschoben wird, wobei das Einfahren des CT-Tisches und das Verschieben der Plattform jeweils mit einer vom Betrag her gleichen Geschwindigkeit durchgeführt werden, sodass der CT-Tisch relativ zu der Standfläche stationär bleibt.

## Claims

1. A computed tomography device (100) for examining a body part of a large animal comprising:
- a computed tomography (CT) device (102) having an annular gantry (104) and a CT table (106) for receiving a body part of a large animal to be examined,
- and a platform (110) on which the CT device (102) is mounted, the gantry (104) being immovable relative to the platform, and
wherein the CT table (106) is horizontally movable relative to the platform and to the gantry and / or the platform is connected such that the CT table (106) in an opening (108) of the annular gantry (104) can be retracted, wherein
the computed tomography device (100) comprises a displacement device, which with the platform (110) connected and adapted to move the platform (110) with the mounted CT device (102) relative to a footprint of a large animal horizontally,
wherein the footprint is located outside the platform, **characterized in that** displacement device is adapted, in at least one operating state, to displace the platform (110) with the CT device (102) in such a way that the CT table (106) remains stationary relative to the footprint during a movement relative to the gantry, by the CT table entering the opening of the annular gantry relative to the platform in a first direction, and the platform is displaced relative to the footprint in a second direction opposite to the first direction, wherein the retraction of the CT table and the displacement of the platform each are performed at an equal speed in terms of amount so that the CT table remains stationary relative to the standing surface.

2. The computed tomography device (100) of claim 1, further comprising a lifting device, wherein the lifting device is configured to vertically raise or lower the platform (110) relative to the footprint of a large animal, such that the CT device mounted on the platform (110) (102) by means of the lifting device and the displacement device relative to the footprint both vertically raised and lowered and can be moved horizontally.

3. Computed tomography device (100) according to claim 1 or 2, wherein the displacement device comprises a rail system (114), which is arranged on a bottom plate (112), and wherein the platform (110) with the mounted CT device (102) on the rail system (114) is arranged so that the bottom plate (112) and the platform (110) are connected by the rail system (114) relative to each other movable.

4. The computed tomography device (100) according to at least one of claims 1 to 3, wherein the displacement device comprises a displacement motor configured to provide a displacement drive for displacing the platform (110) relative to the footprint of a large animal, and wherein the displacement device comprises a threaded spindle and a on the threaded spindle slidably mounted mating thread, wherein the displacement motor for driving the threaded spindle is connected to the threaded spindle, and the mating thread on the platform (110) is arranged, so that during operation of the displacement motor rotation of the threaded spindle in a translational displacement of the platform (110) is converted relative to the base.

5. The computed tomography device (100) of at least one of claims 1 to 4, wherein the CT device comprises a CT drive for motor driven retraction of the CT table (106) into the gantry (104), and wherein a displacement drive and the CT drive a coupling are mechanically coupled to each other, wherein the coupling is designed such that the CT drive is tapped directly and transmitted to the displacement drive.

6. A computed tomography device (100) according to claim 5, wherein the mechanical coupling of the CT drive and the translation drive is configured such that the CT table and the platform (110) move in opposite directions at equal speed with each other in operation.

7. The computed tomography device (100) according to at least one of claims 2 to 6, wherein the platform (110) is arranged with the CT device (102) on a bottom plate (112) and the lifting device comprises a plurality of threaded spindles (120), which in Reference to the bottom plate (112) perpendicular to the platform (110) arranged laterally on the bottom plate (112) and in each case via a displaceably mounted counter-thread (122) to the bottom plate (112) are connected.

8. A computed tomography device according to claim 7, wherein the lifting device comprises at least one lifting motor (124) connected and configured to drive at least one of the plurality of threaded spindles (120) of the lifting device with one of the threaded spindles (120) lowering the platform (110) relative to the footprint of a large animal provide.

9. The computed tomography device according to at least one of claims 2 to 8, wherein the platform is arranged with the CT apparatus on a bottom plate and the lifting device comprises at least one hydraulic pressure cylinder, which is arranged with respect to the bottom plate, that the bottom plate by pressurizing the pressure cylinder can be raised or lowered relative to the base.

10. Computed tomography device (300) according to at least one of claims 1 to 9 having an examination stand, wherein the examination state comprises a holding device (302) and held by the holding device (302) shelf (304), wherein the examination stand is arranged such that the tray (304) is not moved during a displacement of the platform by the displacement device.

11. The computer tomography device (300) of claim 10 including a bottom plate on which the platform is mounted with the mounted CT device, wherein the holding device (302) is attached to the bottom plate so that the holding device (302) and the tray (304) move together with the bottom plate when it is raised or lowered by a lifting device relative to the footprint.

12. The computed tomography device (300) of claim 10 or 11, wherein the tray (304) is mechanically connected to the CT table on the side facing the CT table, and wherein the CT table is decoupled and thereby relative to the rest CT device is freely movable, so that the CT table connected to the tray (304), when the platform mounted CT device is moved together with the platform by the displacement device, by the mechanical connection with the tray relative to the footprint remains stationary during a relative movement to the gantry.

13. A computed tomography examination device (200) comprising a computer tomography device (202) according to at least one of claims 1 to 12, a large animal stand (204) and a floor pit (206) are provided with the computed tomography device (202) disposed in the bottom pit (204) such that at least a portion of the computed tomography device (202) is lower on a lower footprint (204) level is, and where the stand surface (204) located outside the bottom pit (206) adjacent to the side wall (220) of the bottom pit (206) closest to the gantry (210) of the computer tomography device (202).

14. A method of examining a body part of a large animal with a computed tomography device, the computed tomography device comprising:
- a computed tomography (CT) device having an annular gantry and a CT table for receiving a body part of a large animal to be examined, and
- a platform on which the CT device is mounted,
wherein the gantry (104) is immovable relative to the platform,
wherein the CT table (106) relative to the platform and to the gantry horizontally movably connected to the gantry and / or the platform such that
the CT Table (106) can be retracted into an opening (108) of the annular gantry (104),
the method comprising the steps of:
- positioning a large animal on a stand,
- placing a body part to be examined on the CT table,
- moving the platform with the CT device relative to the footprint,
wherein the displacement device is adapted to move in at least one operating state, the platform (110) with the CT device (102) such that the CT table (106) during a relative movement to the gantry remains stationary relative to the footprint,
wherein in the operating condition the CT table moves relative to the platform into the opening of the annular gantry in a first direction, and the platform is displaced in a second direction opposite the first direction relative to the footprint. the retraction of the CT table and moving the platform are each performed at a speed equal in magnitude so that the CT table remains stationary relative to the footprint.

## Revendications

1. Appareil de tomodensitométrie (100) destiné à explorer une partie du corps d'un animal de grande taille, comprenant :
un dispositif de tomodensitométrie (TDM) (102) avec un portique en forme d'anneau (104) et une table TDM (106) pour recevoir une partie du corps d'un animal de grande taille à explorer, et
une plate-forme (110) sur lequel le dispositif TDM (102) est monté,
dans lequel le portique (104) est immobile par rapport à la plate-forme, et
dans lequel la table TDM (106) est mobile horizontalement par rapport à la plate-forme et au portique et est relié au portique et/ou à la plate-forme de manière à ce que la table TDM (106) puisse être introduite dans une ouverture (108) du portique en forme d'anneau (104),
l'appareil de tomodensitométrie (100) comprenant un dispositif de déplacement qui est relié à la plate-forme (110) et qui est conçu pour déplacer la plate-forme (110), avec le dispositif TDM (102) monté, horizontalement par rapport à une surface d'appui d'un animal de grande taille,
la surface d'appui étant à l'extérieur de la plate-forme, **caractérisé en ce que**
le dispositif de déplacement est conçu pour déplacer la plate-forme (110) avec le dispositif TDM (102) dans au moins un état de fonctionnement de telle sorte que la table TDM (106) reste immobile par rapport à la surface d'appui pendant un mouvement relatif par rapport au portique, **en ce que** la table TDM se déplace par rapport à la plate-forme dans l'ouverture du portique en forme d'anneau dans une première direction, et la plate-forme est déplacée dans une seconde direction, qui est opposée à la première direction, par rapport à la surface d'appui, dans lequel l'introduction de la table TDM et le déplacement de la plate-forme peut être effectué dans chaque cas avec la même vitesse en fonction du montant de sorte que la table TDM reste stationnaire par rapport à la surface d'appui.

2. Appareil de tomodensitométrie (100) selon la revendication 1, qui comporte en outre un dispositif de levage, dans lequel le dispositif de levage est conçu pour élever ou abaisser verticalement la plate-forme (110) par rapport à la surface d'appui d'un animal de grande taille, de sorte que le dispositif TDM (102) monté sur la plate-forme (110) peut être relevé et abaissé à la fois verticalement et horizontalement par rapport à la surface d'appui au moyen du dispositif de levage et du dispositif de déplacement.

3. Appareil de tomodensitométrie (100) selon la revendication 1 ou 2, dans lequel le dispositif de déplacement comprend un système de rails (114) qui est disposé sur une plaque de base (112), et dans lequel la plate-forme (110) avec le dispositif TDM (102) monté est agencé sur le système de rails (114) de telle sorte que la plaque de base (112) et la plate-forme (110) sont reliées ensemble de manière mobile relativement l'une vers l'autre par le système de rails (114).

4. Appareil de tomodensitométrie (100) selon au moins l'une des revendications 1 à 3, dans lequel le dispositif de déplacement comprend un moteur de déplacement qui est conçu pour fournir un entraînement de déplacement pour déplacer la plate-forme (110) par rapport à la surface d'appui d'un animal de grande taille, et dans lequel le dispositif de déplacement présente une broche filetée et comprend un filetage d'accouplement monté de manière déplaçable sur la broche filetée, le moteur de déplacement pour entraîner la broche filetée étant connecté à la broche filetée, et le filetage d'accouplement étant agencé sur la plate-forme (110), de sorte que lorsque le moteur de déplacement est en fonctionnement, une rotation de la broche filetée est transformée en un déplacement de translation de la plate-forme (110) par rapport à la surface d'appui.

5. Appareil de tomodensitométrie (100) selon au moins l'une des revendications 1 à 4, dans lequel le dispositif TDM comprend un entraînement TDM pour l'introduction motorisée de la table TDM (106) dans le portique (104), et dans lequel un entraînement de déplacement et l'entraînement TDM sont couplés mécaniquement l'un à l'autre, l'accouplement étant conçu de telle sorte que l'entraînement TDM soit prélevé directement et transmis à l'entraînement de déplacement.

6. Appareil de tomodensitométrie (100) selon la revendication 5, dans lequel le couplage mécanique de l'entraînement TDM et de l'entraînement de déplacement est conçu de telle sorte que la table TDM et la plate-forme (110) soient déplacées en fonction du montant à la même vitesse dans des sens opposés.

7. Appareil de tomodensitométrie (100) selon au moins l'une des revendications 2 à 6, dans lequel la plate-forme (110) avec le dispositif TDM (102) est disposée sur une plaque de base (112) et le dispositif de levage comprend une pluralité de broches filetées (120), qui par rapport à la plaque de base (112), sont disposées perpendiculairement en direction de la plate-forme (110) du côté de la plaque de base (112) et sont respectivement reliées à la plaque de base (112) via un contre-filetage (122) monté de façon déplaçable.

8. Appareil de tomodensitométrie selon la revendication 7, dans lequel le dispositif de levage comprend au moins un moteur de levage (124) qui, pour entraîner au moins l'une de la pluralité de broches filetées (120) du dispositif de levage, est connecté avec l'une des broches filetées (120), et est conçu pour assurer un entraînement de levage pour la montée ou la descente de la plate-forme (110) par rapport à la surface d'appui d'un animal de grande taille.

9. Appareil de tomodensitométrie selon au moins l'une des revendications 2 à 8, dans lequel la plate-forme avec le dispositif TDM est agencée sur une plaque de base et le dispositif de levage comprend au moins un vérin hydraulique de pression qui est agencé par rapport à la plaque de base de sorte que, par application de pression au vérin, la plaque de base peut être relevé ou abaissé par rapport à la surface d'appui.

10. Appareil de tomodensitométrie (300) selon au moins l'une des revendications 1 à 9, qui comporte un banc d'examen, dans lequel le banc d'examen comprend un dispositif de maintien (302) et un plateau (304) maintenu par le dispositif de maintien (302), le banc d'examen étant agencé de telle sorte que le plateau (304) n'accompagne pas le déplacement de la plate-forme, déplacée par le dispositif de déplacement.

11. Appareil de tomodensitométrie (300) selon la revendication 10 qui comprend une plaque de base sur laquelle est disposée la plate-forme avec le dispositif TDM monté, dans lequel le dispositif de maintien (302) est fixé à la plaque de base de sorte que le dispositif de maintien (302) et le plateau (304) se déplacent avec la plaque de base lorsqu'elle est soulevée ou abaissée par rapport à la surface d'appui par un dispositif de levage.

12. Appareil de tomodensitométrie (300) selon la revendication 10 ou 11, dans lequel le plateau (304) est relié mécaniquement à la table TDM du côté faisant face à la table TDM, et dans lequel la table TDM est découplée et donc par rapport au reste du dispositif TDM est librement déplaçable, de sorte que la table TDM connectée au plateau (304) reste stationnaire lorsque le dispositif TDM monté sur la plate-forme est déplacé avec la plate-forme par le dispositif de déplacement, par le biais de la connexion mécanique avec le plateau par rapport à la surface d'appui pendant un mouvement relatif au portique.

13. Agencement d'examen par tomodensitométrie (200) comprenant un appareil de tomodensitométrie (202) selon au moins l'une des revendications 1 à 12, une surface d'appui (204) pour un animal de grande taille et une fosse au sol (206), dans lequel l'appareil de tomodensitométrie (202) est dans la fosse au sol (204) de telle sorte qu'au moins une partie de l'appareil de tomodensitométrie (202) est à un niveau inférieur par rapport à la surface d'appui (204), et la surface d'appui (204) est à l'extérieur de la fosse au sol (206) et se trouve adjacente à la paroi latérale (220) de la fosse au sol (206) qui est la plus proche du portique (210) de l'appareil de tomodensitométrie (202).

14. Procédé d'examen d'une partie du corps d'un animal de grande taille avec un appareil de tomodensitométrie, l'appareil de tomodensitométrie comportant :
un dispositif de tomodensitométrie (TDM) avec un portique en forme d'anneau et une table TDM pour recevoir une partie du corps d'un animal de grande taille à explorer, et
une plate-forme sur laquelle le dispositif de tomodensitométrie est monté,
dans lequel le portique (104) est immobile par rapport à la plate-forme,
dans lequel la table TDM (106) en étant mobile horizontalement par rapport à la plate-forme et au portique est connectée au portique et/ou à la plate-forme de manière à pouvoir se déplacer de telle sorte que
la table TDM (106) peut être introduite dans une ouverture (108) du portique en forme d'anneau (104), le procédé comprenant les étapes consistant à :
positionner un animal de grande taille sur une surface d'appui,
placer une partie du corps à explorer sur la table TDM,
déplacer la plate-forme avec le dispositif TDM par rapport à la surface d'appui, dans lequel le dispositif de déplacement est réalisé de sorte que, dans au moins un état de fonctionnement, la plate-forme (110) avec le dispositif TDM (102) est déplacée de telle manière que la table TDM (106) reste stationnaire pendant un mouvement relatif au portique par rapport à la surface d'appui,
dans lequel, à l'état de fonctionnement, la table TDM se déplace par rapport à la plate-forme dans l'ouverture du portique en forme d'anneau dans une première direction, et la plate-forme est déplacée dans une seconde direction, qui est opposée à la première direction, par rapport à la surface d'appui, dans lequel l'introduction de la table TDM et le déplacement de la plate-forme peut être effectué dans chaque cas avec la même vitesse en fonction du montant de sorte que la table TDM reste stationnaire par rapport à la surface d'appui.
